Europäisches Patentamt

European Patent Office

Office européen des brevets

(19)

(11) Numéro de publication : **0 309 533 B1**

(12)                 FASCICULE DE BREVET EUROPEEN

(45) Date de publication du fascicule du brevet :
02.10.91 Bulletin 91/40

(51) Int. Cl.⁵ : **A61K 31/085, C07C 43/23**

(21) Numéro de dépôt : 88903278.5

(22) Date de dépôt : 08.04.88

(86) Numéro de dépôt international :
PCT/FR88/00173

(87) Numéro de publication internationale :
WO 88/07856 20.10.88 Gazette 88/23

(54) BUTYL HYDROXYANISOLES POUR LE TRAITEMENT DES MALADIES A RETRO-VIRUS.

(30) Priorité : 10.04.87 FR 8705088

(43) Date de publication de la demande :
05.04.89 Bulletin 89/14

(45) Mention de la délivrance du brevet :
02.10.91 Bulletin 91/40

(84) Etats contractants désignés :
AT BE CH DE FR GB IT LI LU NL SE

(56) Documents cités :
Antibacteril Agents and Chemotherapy, July
1976, Vol. 10, no. 1, pp. 96-101
CHEMICAL ABSTRACTS, vol. 85 no. 13, 27
sept. 1976, (Columbus Ohio, US) P. Wanda et
al.: "Inactivation of the enveloped bacteriophage 06 by butylated hydroxytolueme and
butylated hydroxyanisole"

(56) Documents cités :
Chemical Abstracts, vol. 77 no. 15, 9 October
1972,(Columbus, Ohio, US) L.W. Wattenberg :
"Inhibition of carcinogenic and toxic effects of
polycyclic hydrocarbons by phenolic antioxidants ans ethoxyquin", see page 86, abstract
9753e, J. Nat. Cancer Inst. 1972, 48 (5), 1425-30
Chemical Abstracts, vol. 82, no. 23, 9 June
1975, Columbus, Ohio, USW. Snipes et al.:
"Butylated hydroxytoluene inactivated in
lipid-containing viruses", see page 86, abstract 149903x, Science 1975, 188 (4183), 64-6

(73) Titulaire : ROUSSEL-UCLAF
35, boulevard des Invalides
F-75007 Paris (FR)

(72) Inventeur : MARCEL, Georges
258, boulevard Saint-Germain
F-75007 Paris (FR)

(74) Mandataire : Fritel, Hubert et al
Département des Brevets ROUSSEL UCLAF
B.P. no 9
F-93230 Romainville (FR)

## Description

La présente invention a pour objet l'utilisation des composés chimiques constitués par les butyl hydroxyanisoles et leurs sels pour la fabrication de médicaments destinés au traitement des maladies rétro-virus et, notamment, du Sida. Les composés impliqués dans l'invention, dénommés composés (I), comprennent notamment les ter-butyl hydroxyanisoles, comme le 3-ter butyl 4-hydroxyanisole (II), le 2-ter butyl 4-hydroxyanisole (III) et le mélange (IV) en proportion variable de ces 2 produits, mélange quelquefois désigné sous le terme B.H.A.

L'utilisation des composés (I) permet le traitement des maladies provoquées par les rétro-virus, par exemple le traitement du Sida, il peut s'agir alors d'un traitement prophylactique ou d'un traitement curatif.

Les composés impliqués dans l'invention et leurs sels, tels que les sels alcalins, sont des composés chimiques, connus depuis longtemps. Les composés (II) et (III) et leur mélange, notamment le composé (IV) dénommé B.H.A, possèdent de très intéressantes propriétés anti-oxydantes, tout en étant aussi peu toxiques que possible vis-à-vis de l'homme et des animaux. En particulier, ces études ont montré la non-nocivité de doses orales allant chez le singe Rhesus jusqu'à 500 mg/kg/j pendant 1 mois, chez le singe Cynomolgus jusqu'à 400 mg/kg/j pendant 3 mois, chez le porc jusqu'à 400 mg/kg/j pendant 4 mois, chez le chien Beagle jusqu'à 220 mg/kg/j pendant 6 mois. Ils sont de ce fait largement utilisés, tel le B.H.A, comme additifs alimentaires pour la conservation des graisses et des huiles comestibles et de nombreux autres aliments à usage humain. Ils sont également utilisés comme conservateurs dans la fabrication des médicaments. Toutefois, il apparaît que ces composés n'ont jamais été utilisés en tant que principes actifs dans un but thérapeutique à l'égard de l'homme ou des animaux.

On sait, par ailleurs, que les rétro-virus, dont fait partie le virus du Sida (H.I.V ou H.T.L.V III ou L.A.V.) sont des virus à enveloppe. Or, d'après les connaissances actuelles, il semblerait que le virus du Sida soit pathogène du fait d'un mécanisme en 2 temps :

— dans un premier temps, l'enveloppe du rétro-virus adhère à un récepteur de la membrane des cellules telles les lymphocytes T4.

— dans un second temps, le rétro-virus pénètre la cellule et, du fait de la transcriptase inverse, son RNA est transcrit en DNA, lequel s'intègre au génome de la cellule infectée, qui, dès lors, est susceptible d'assurer la multiplication du virus.

Jusqu'à maintenant les recherches thérapeutiques en matière de Sida ont essentiellement porté sur le second temps du mécanisme décrit plus haut, en faisant appel en particulier aux inhibiteurs de la transcriptase inverse ou d'autres enzymes impliquées dans la réplication, la transcription et la traduction des acides nucléiques.

Il ne semble pas, au contraire, que jusqu'ici le premier temps du mécanisme supposé d'action du rétrovirus du Sida, ait donné lieu à des études approfondies. C'est précisément pour tenter de développer une thérapeutique prophylactique et/ou curative du Sida que l'auteur de la présente invention a porté son attention sur le premier temps de ce mécanisme, c'est-à-dire sur l'adhésion de l'enveloppe virale au récepteur cellulaire, son mécanisme, son substrat biochimique et les moyens de le modifier. L'importance de cette enveloppe est attestée par la taille de l'unité de transcription (ou gène, ou "open reading frame" = orf) correspondante. En effet le H.I.V. possède au moins 6 orfs : le plus volumineux (ou "pol") code pour la transcriptase inverse, le suivant en volume (ou "env") code pour une glycoprotéine précurseur (g Pr 160) qui est clivée en glycoprotéine externe (gp 120) et glycoprotéine transmembranaire (gp 41), de dimensions inhabituelles.

Il a été alors montré, et c'est ce qui fait l'objet essentiel de la présente invention, que les composés (I) tels que définis plus haut présentent des propriétés remarquables et surprenantes ayant pour effet de supprimer, réduire ou modifier l'infectivité du virus du Sida. Cet effet est dû très vraisemblablement à l'altération de l'enveloppe de ce rétro-virus et, dans cette hypothèse, pourrait donc intervenir au niveau du premier temps du mécanisme évoqué ci-dessus.

Un effet voisin a certes été envisagé récemment pour le 2,6-diter-butyl-4-méthylphénol ou B.H.T. ; il ne s'agissait là toutefois que d'une hypothèse de travail. Par ailleurs, les études menées antérieurement sur le B.H.A. et le B.H.T., tout particulièrement sur ce dernier composé, concernaient des virus n'appartenant pas à la catégorie des rétro-virus et suggéraient avant tout des potentialités d'utilisation. Par la suite, l'application du B.H.T. à la prophylaxie de la maladie de Newcastle des poules et au traitement curatif de l'herpès fut essayée mais se révéla très décevante (cf Snipes, Person, Keith, Cupp Science (1975), 187, 64 ; Wanda, Cupp, Snipes et coll. Antimicrobial Agents and Chemotherapy (1976), vol. 10, page 96 ; Brugh M. Science. (1977), vol. 197 pages 1291-1292 ; Freeman D.F., Wenerstrom G., Spruance S.L. Clin. Pharmacol. Therap. (1905), vol. 38, pages 56-59). La présente invention a donc pour objet l'utilisation des composés (I) en tant qu'agents antiviraux et/ou anti-infectants ou antiseptiques.

L'activité antivirale des composés (I) de l'invention, notamment du composé (II), a été mise en évidence sur des virus H.I.V. provenant d'un surnageant de culture de cellules continuellement productrices de ce virus. Elle est illustrée plus loin dans la partie expérimentale.

En raison de leurs propriétés antivirales et/ou anti-infectantes ainsi que de leur innocuité reconnue à l'égard de l'homme, les composés (I) selon l'invention trouvent leur emploi comme médicaments par exemple dans le traitement prophylactique ou curatif des maladies à rétro-virus notamment du Sida. Pour une telle utilisation thérapeutique, on prépare, selon les méthodes usuelles, des compositions pharmaceutiques, objet également de l'invention, comportant comme principe actif un composé (I) tel que le composé (II) ou un mélange de composés (I) et un excipient pharmaceutique inerte.

Parmi les formes galéniques habituelles propres à assurer au mieux un traitement prophylactique, on peut citer les crèmes, pommades, gels, lotions, poudres, émulsions, aérosols. Parmi celles-ci, les aérosols génitaux, les aérosols oraux, les gels vaginaux avec applicateur sont tout particulièrement recommandés. L'excipient utilisé dans ces compositions sera de préférence un excipient visqueux permettant la rémanence du principe actif au niveau des sites potentiels d'inoculation. Parmi de tels excipients, on peut citer :

(A) les huiles neutres du type triglycérides d'acides gras saturés d'origine végétale en $C_8$-$C_{12}$, avec ou sans ions $Ca^{++}$

(B) l'éthanol à 90-97%, renfermant 0,10 à 0,20% d'esters de cellulose, avec ou sans ions $Ca^{++}$.

La dose de composés (I) dans ces compositions peut dépendre de la forme utilisée. Elle sera notamment comprise entre 0,01% et 1% de principe actif, de préférence de l'ordre de 0,02%. La dose maximale quotidienne de principe actif, variable selon le sujet à traiter, est de l'ordre de 30 mg, ce qui peut correspondre par exemple pour un aérosol oral à une trentaine de vaporisations. Dans le cadre également de la présente invention, en raison de l'aptitude des composés (I), tels que les composés (II) et (IV), à inactiver complètement le pouvoir infectieux du virus HIV 1, et étant donné l'inocuité et la maniabilité de ces molécules, attestées par des études toxicologiques et pharmacocinétiques, les composés (I) de l'invention, notamment le composé (II), doivent avoir leur place dans le traitement curatif des infections provoquées par le virus HIV 1 et les virus apparentés. Cette utilisation thérapeutique peut s'adresser à la simple séropositivité, au syndrome dénommé ARC (AIDS related complex) ou au Sida-maladie. Dans un tel traitement curatif, le composé (II), notamment, pourra être administré per os ou par voie parentérale, en particulier par voie intraveineuse lente.

La posologie peut varier en fonction de la voie d'administration, de l'affection traitée et du sujet en cause. Par exemple, elle variera entre 50 et 100 mg/kg/jour per os et entre 1 à 10 mg/kg/jour par voie parentérale.

Les composés de l'invention, tels que (II), pourront être employés seuls ou en mélange, ou bien même en association avec d'autres médicaments présentés pour le traitement curatif du Sida, médicaments qui n'agissent pas sur le même phénomène infectieux que les composés (I).

Parmi les associations possibles, citons par exemple l'association avec l'azidothymidine, la didéoxycitidine, l'HPA-23 et l'AL-721.

La présente invention a donc pour objet l'utilisation des composés (I) pour la fabrication de médicaments destinés au traitement prophylactique ou curatif des maladies à rétro-virus telles que le Sida, ainsi que pour la fabrication de médicaments ayant une activité antivirale et/ou anti-infectante sur le virus du Sida et les virus apparentés.

La propriété mise en évidence pour les composés (I) d'agir sur l'infectivité du virus responsable du Sida, permet par ailleurs l'utilisation des composés de l'invention, notamment des composés (II) et (IV), pour la préparation de vaccins anti-Sida selon les méthodes usuelles de préparation des vaccins. Dans cette utilisation, objet également de la présente invention, les composés (I) agissent comme agents modificateurs de l'infectivité du rétrovirus du Sida tout en n'en détruisant pas les caractéristiques physiques et antigéniques. Ils permettent par cette action de préparer un rétro-virus à infectivité atténuée mais à anti-génicité persistante pouvant être ainsi à l'origine d'un vaccin.

Egalement, Compte tenu des propriétés des composés (I), notamment de leurs propriétés anti-infectantes et/ou antiseptiques, l'invention a encore pour objet ces composés en tant que produits anti-infectants et/ou antiseptiques ainsi que les compositions renfermant ces produits. Ces compositions sont destinées à combattre localement les rétro-virus tels que le virus du Sida en supprimant ou réduisant leur infectivité. De telles compositions, destinées à un usage humain, au vu de leur forme et destination, ne seraient pas ou pourraient ne pas être considérées comme des compositions pharmaceutiques. Parmi les formes possibles d'utilisation, citons en particulier les gommes à mâcher, les pommades pour lèvres, les savons, les laits, les gels de douche, les bains de bouche. Ces compositions sont préparées selon les méthodes usuelles.

Ces produits anti-infectants et/ou antiseptiques tels que définis ci-dessus et des compositions les renfermant sont aptes aussi à être utilisés en application sur des objets ou dans des locaux susceptibles d'être infectés par le virus du Sida.

Parmi les compositions possibles pour un tel usage, on peut citer les aérosols, cires, enduits.

Les exemples suivants illustrent l'invention sans toutefois la limiter.

## Exemple 1

Une solution mère de 3-ter-butyl 4-hydroxyanisole (II) à 0,5 M dans l'éthanol à 95% a été préparée et sa compatibilité testée avec le milieu de culture RPMI 1640 additionné de sérum de veau foetal (10%), d'interleukine II (10%) et de sérum anti-interféron humain.

Il est vérifié que les concentrations $10^{-5}$ à $10^{-3}$ M de composé (II) ne sont pas toxiques pour les lymphocytes T directement et après lavage.

Des virus H.I.V. (provenant d'un surnageant de cellules continuellement productrices de virus H.I.V. de titre supérieur en activité transcriptase inverse à $10^5$ cpm/ml) incubés 5, 10, 30 ou 60 minutes à 37° avec des concentrations de $10^{-5}$ à $10^{-3}$ M de composé (II), sont ensuite mis en contact avec des lymphocytes T d'un donneur normal (stimulés par PHA-P pendant 3 jours, puis cultivés en milieu additionné d'IL II et de sérum anti-interféron humain). L'infectivité de ces préparations est déterminée en suivant la production virale (activité transcriptase inverse) dans les surnageants de culture de cellules T infectées, à chaque passage cellulaire (tous les 3 ou 4 jours), pendant un mois. L'infectivité des virus H.I.V. est maintenue pour des témoins non traités ou traités par des concentrations de composé (II) $10^{-5}$ ou $10^{-4}$ M. Elle disparaît lorsqu'on utilise le composé (II) à la concentration finale de $10^{-3}$ M.

Ainsi, à cette concentration, une incubation minimum, de 30 minutes, à l'abri de la lumière et à une température de 37°C, du composé (II) avec le virus HIV 1 suffit à inactiver complètement le pouvoir infectieux du virus du Sida.

Une étude comparative effectuée avec la 2,6-diter-butyl-4-méthyl phénol ou B.H.T. donne le résultat suivant à J + 4 :

```
composé (II) :        355
B.H.T.        :    113.624
```

(les chiffres représentent l'activité transcriptase inverse, c'est-à-dire la production virale, exprimée en c.p.m./ml). Elle montre également que l'activité anti-Sida n'appartient pas à un dérivé proche.

Exemple 2 : aérosol génital

L'aérosol génital est constitué par une solution $10^{-3}$ M de composé (II) dans un excipient du type (A) ou (B).

Cette solution visqueuse est destinée à permettre de recouvrir les régions génitales susceptibles d'être contaminées. Une vaporisation correspondra à 5 ml soit 0,9 mg de principe actif, ce qui permet 30 vaporisations par jour sans dépasser la dose maximale admise pour la voie orale.

Exemple 3 : aérosol oral

L'aérosol oral est constitué par une solution mentholée $10^{-3}$ M de composé (II) dans un excipient du type (A) ou (B).

Cette solution visqueuse est destinée à permettre de recouvrir la muqueuse endo-buccale. Une vaporisation correspondra également à 5 ml soit 0,9 mg de principe actif.

Exemple 4 : gel vaginal avec applicateur

Le gel vaginal est constitué par un gel $10^{-3}$ M de composé (II) destiné à permettre de recouvrir la muqueuse vaginale. La dose unitaire est de 5 ml soit 0,9 mg de principe actif.

## Revendications

1. Utilisation des composés constitués par les butyl hydroxyanisoles et leurs sels pour la fabrication de médicaments destinés au traitement des maladies à rétro-virus.

2. Utilisation, selon la revendication 1, du 3-terbutyl 4-hydroxyanisole.

3. Utilisation, selon la revendication 1, d'un mélange de 3-terbutyl 4-hydroxyanisole et de 2-terbutyl 4-hydroxyanisole.

4. Utilisation des composés, selon la revendication 1, 2 ou 3, pour la fabrication de médicaments destinés au traitement du Sida.

5. Utilisation des composés, selon la revendication 1, 2 ou 3, pour la fabrication de médicaments ayant une activité antivirale sur les rétro-virus.

6. Utilisation, selon la revendication 5, du 3-terbutyl 4-hydroxyanisole pour la fabrication d'un médicament ayant une activité antivirale sur le virus du Sida.

7. Utilisation des composés, selon la revendication 1, 2 ou 3, pour la fabrication de médicaments ayant une activité anti-infectante sur le virus du Sida.

8. Utilisation des composés constitués par les butyl hydroxyanisoles et leurs sels pour la fabrication de vaccins anti-Sida.

9. Utilisation, selon la revendication 8, du 3-terbutyl 4-hydroxyanisole.

10. Utilisation des composés constitués par les butyl hydroxyanisoles et leurs sels pour la fabrication d'agents modificateurs de l'infectivité du rétro-virus du Sida.

11. Utilisation, selon la revendication 10, du 3-terbutyl 4-hydroxyanisole.

12. Utilisation, selon la revendication 8, 9, 10 ou 11, des composés y mentionnés pour la préparation de rétro-virus à infectivité atténuée mais anti-génécité persistante.

## Claims

1. Use of compounds constituted by butyl hydroxyanisoles and their salts for the manufacture of medicaments intended for the treatment of retroviral illnesses.

2. Use, according to claim 1, of 3-terbutyl-4-hydroxyanisole.

3. Use, according to claim 1, of a mixture of 3-terbutyl-4-hydroxyanisole and 2-terbutyl-4-hydroxyanisole.

4. Use of compounds, according to claim 1, 2 or 3, for the manufacture of medicaments intended for the treatment of Aids.

5. Use of compounds, according to claim 1, 2 or 3, for the manufacture of medicaments having an antiviral activity on retroviruses.

6. Use, according to claim 5, of 3-terbutyl-4-hydroxyanisole for the manufacture of a medicament having an antiviral activity on the Aids virus.

7. Use of compounds, according to claim 1, 2 or 3, for the manufacture of medicaments having an antiinfecting activity on the Aids virus.

8. Use of compounds constituted by butyl hydroxyanisoles and their salts for the manufacture of anti-Aids vaccines.

9. Use, according to claim 8, of 3-terbutyl-4-hydroxyanisole.

10. Use of compounds constituted by butyl hydroxyanisoles and their salts for the manufacture of modifying agents of the infectivity of the Aids retrovirus.

11. Use, according to claim 10, of 3-terbutyl-4-hydroxyanisole.

12. Use, according to claim 8, 9, 10 or 11, of the compounds mentioned therein for the preparation of a retrovirus with attenuated infectivity but persistent anti-genicity.

## Patentansprüche

1. Verwendung der aus den Butylhydroxyanisolen und deren Salzen bestehenden Verbindungen für die Herstellung von Arzneimitteln zur Behandlung von Erkrankungen mit Retroviren.

2. Verwendung gemäß Anspruch 1 von 3-tert.-Butyl-4-hydroxyanisol.

3. Verwendung gemäß Anspruch 1 eines Gemisches von 3-tert.-Butyl-4-hydroxyanisol und 2-tert.-Butyl-4-hydroxyanisol.

4. Verwendung der Verbindungen gemäß Anspruch 1, 2 oder 3, für die Herstellung von Arzneimitteln zur Behandlung von Sida.

5. Verwendung der Verbindungen gemäß Anspruch 1, 2 oder 3 für die Herstellung von Arzneimitteln mit einer antiviralen Aktivität gegenüber Retroviren.

6. Verwendung gemäß Anspruch 5 von 3-tert.-Butyl-4-hydroxyanisol für die Herstellung eines Arzneimittels mit einer antiviralen Aktivität gegenüber dem Sida-Virus.

7. Verwendung der Verbindungen gemäß Anspruch 1, 2 oder 3 für die Herstellung von Arzneimitteln mit einer anti-infizierenden Aktivität gegenüber dem Sida-Virus.

8. Verwendung der aus den Butylhydroxyanisolen und deren Salzen bestehenden Verbindungen für die Herstellung von Anti-Sida-Vakzinen.

9. Verwendung gemäß Anspruch 8 von 3-tert.-Butyl-4-hydroxyanisol.

10. Verwendung der aus den Butylhydroxyanisolen und deren Salzen bestehenden Verbindungen für die Herstellung von die Infektivität des Sida-Retrovirus modifizierenden Mitteln.

11. Verwendung gemäß Anspruch 10 von 3-tert.-Butyl-4-hydroxyanisol.

12. Verwendung gemäß Anspruch 8, 9 10 oder 11 der dort genannten Verbindungen für die Herstellung von Retroviren mit abgeschwächter Infektivität, jedoch mit fortbestehender Antigenezität.